# EUROPÄISCHE PATENTSCHRIFT

(11) **EP 1 250 864 B1**
(45) Veröffentlichungstag und Bekanntmachung des Hinweises auf die Patenterteilung: **16.05.2007**
(21) Anmeldenummer: 02003567.1
(22) Anmeldetag: 15.02.2002
(51) Int. Cl.: A45D 34/04

(54) **Applikationsvorrichtung**
Application device
Dispositif d'application

(30) Priorität: 20.04.2001 DE 10119480
(43) Veröffentlichungstag der Anmeldung: 23.10.2002
(73) Patentinhaber: Firma Ivoclar Vivadent AG, 9494 Schaan (LI)
(72) Erfinder: Müller, Frank, 6800 Feldkirch (AT)
(74) Vertreter: Baronetzky, Klaus

(56) Entgegenhaltungen:
- WO-A-01/23262
- US-A- 4 206 843
- US-A- 5 573 340

## Beschreibung

Die Erfindung betrifft eine Applikationsvorrichtung, gemäß dem Oberbegriff von Anspruch 1.

Aus der US-PS 5,573,340 ist ein Container für die Verteilung eines flüssigen oder pulverförmigen Produktes bekannt, bei dem ein Schwamm mit einer Flüssigkeit benetzbar ist.

Für das Aufbringen von Substanzen ist es seit langem bekannt, einen ersten Bestandteil in einer ersten Kammer und einen weiteren Bestandteil in einer zweiten Kammer aufzubewahren und durch Druck von außen eine Sollbruchstelle, die die beiden Bestandteile trennt, zu brechen und hierdurch die Bestandteile zu der Substanz zu vermischen. Hierzu sei beispielsweise auf die DE-AS 2 024 402 verwiesen.

Applikationsvorrichtungen mit Applikationselementen sind ebenfalls seit langem bekannt, wozu beispielhaft auf die DE-GM 92 189 49 verwiesen wird. Über seitliche Löcher in einer Innenhülse kann eine Fluidverbindung zwischen dem Innenraum der Innenhülse und dem Innenraum der Außenhülse erzeugt werden. Diese Lösung ist für den Kosmetikbereich geeignet. Wenn es darum geht, dass die Bestandteile im Innenraum der Innenhülse und im Innenraum der Außenhülse erzeugt werden. Diese Lösung ist für den Kosmetikbereich geeignet. Wenn es darum geht, dass die Bestandteile im Innenraum der Innenhülse und im Innenraum der Außenhülse voneinander getrennt gelagert werden müssen, ist dies Lösung jedoch weniger geeignet.

Ferner ist aus der DE-OS 44 158 54 eine Misch- und Applikationskapsel für Dentalzwecke bekannt. Bei dieser Lösung werden die Bestandteile dadurch vereinigt, dass über ein spitzes Element eine Folie zwischen den Bestandteilen durchstochen wird.

Zahlreiche weitere Applikationsvorrichtungen sind bekannt geworden. So ist es beispielsweise vorgeschlagen worden, ein Applikationselement in einer Flüssigkeit eingetaucht zu halten und durch Niederdrücken des Behälters für diese Flüssigkeit nach der Art eines Überlaufs eine weitere Flüssigkeit einströmen zu lassen, um die aufzubringende Substanz bereitzustellen. Diese Lösung bedarf allerdings zweier getrennter Abdichtsysteme, so dass ein nicht unbeachtlicher Aufwand erforderlich ist. Eine Vielzahl von Dichtlinien bedingt allerdings auch eine entsprechend große Anfälligkeit und Lagerinstabilität.

Ferner ist es in der deutschen Patentanmeldung 199 56 705.0 vorgeschlagen worden, ein Applikationselement mit einem Benetzungskörper in einer Innenhülse teilweise aufzunehmen und die Innenhülse in einer Außenhülse aufzunehmen. Durch Druck auf das Applikationselement soll eine Flüssigkeitsverbindung zwischen dem Innenraum und der Innenhülse und dem Innenraum der Außenhülse erzeugt werden. Diese Lösung bietet im Grunde eine gute und einfache Abdichtung der beiden Bestandteile zur Bereitstellung der Substanz. Allerdings muß die Außenhülse zur Bereitstellung der Dichtlippen aus einem etwas elastischem Material hergestellt werden, damit ein flüssigkeitsdichtes Anliegen der Dichtlippe zwischen Innenhülse und Außenhülse gewährleistet ist. Andererseits ist es wichtig, dass die in den Innenraum der Außenhülse aufgenommene Substanz auch über eine längere Zeit sicher gelagert werden kann. Um dies zu erzielen, ist eine recht große Wandstärke vorgesehen.

Teilweise werden aber auch Lösungsmittel wie Aceton, Äthylalkohol, usw. für die Bestandteile der Substanz eingesetzt. Derartige Lösungsmittel weisen einen ausgesprochen hohen Dampfdruck auf und diffundieren auch dann durch die Außenhülse, wenn sie eine recht große Wandstärke aufweist.

Zwar ist es an sich bekannt geworden, einem hohem Dampfdruck durch das Aufbringen zusätzlicher Schichten, wie beispielsweise einer metallischen Beschichtung, zu begegnen. Problem hierbei ist, dass zweischichtige Materialien bei deren Verarbeitung und der Entsorgung problematischer sind und auch keine vollständige Dampfsperre möglich ist. Zwar könnte die metallische Beschichtung auch innen aufgebracht werden. Dann erstreckt sie sich aber über die dortigen Dichtlippen hinweg, so dass die Dichtwirkung vermindert ist. Zudem verteuert die Bereitstellung einer metallischen Beschichtung die Herstellung der Applikationsvorrichtung, wobei die Herstellungskosten gerade bei Realisierung einer Single-Dose-Einheit wichtig sind.

Daher liegt der Erfindung die Aufgabe zugrunde, eine preisgünstig herzustellende Applikationsvorrichtung für das Auftragen einer Substanz gemäß dem Oberbegriff von Anspruch 1 zu schaffen, die auch für Lagerzeiten von mehr als einem Jahr und auch für Substanzen mit hohem Dampfdruck geeignet ist.

Diese Aufgabe wird erfindungsgemäß durch Anspruch 1 gelöst. Vorteilhafte Weiterbildungern ergeben sich aus den Unteransprüchen.

Erfindungsgemäß besonders günstig ist es, dass die Realisierung der Innenhülse aus einem weicheren Material es erlaubt, die Dichtfunktion durch die Innenhülse selbst sicherzustellen und die Außenhülse aus einem spröden und hochdichten Material zu realisieren. Diese Lösung ist preisgünstig, denn die Wandstärke der Außenhülse kann so reduziert werden und dennoch die Lagerstabilität aufgrund der verbesserten Dampfdruckdichtheit bereitgestellt werden.

Die erfindungsgemäß erwünschte Weichheit und Verformbarkeit der Innenhülse läßt sich sowohl materialspezifisch als auch durch die Gestaltung der Innenhülse bereitstellen. Wenn ein Verformungselement eine einseitig wirkende radiale Kraft auf den Boden der Innenhülse oder einen bodennahen Bereich der Innenhülse ausübt, kollabiert die Innenhülse dort mindestens teilweise, so dass die Abdichtung zwischen Innenhülse und Außenhülse in der erwünschten Weise verloren geht.

Erfindungsgemäß ist es bevorzugt, wenn die Dichtlippe sich ringförmig im bodennahen Bereich an der Innenhülse nach außen erstreckt. Die Wand der Außenhülse kann damit innen bis zum Verformungselement hin völlig glatt ausgebildet sein.

Erindungsgemäß besonders günstig ist es, wenn der Boden der Innenhülse recht dünn ausgebildet ist. Durch den einseitigen und massiven Keil, der das Verformungselement bilden kann, wird bei einem entsprechend dünnen Boden die Innenhülse verformt, auch wenn die Materialeigenschaften von Innenhülse und Außenhülse sich nur um Weniges unterscheiden. Insofern ist die Formunlierung weicher auch in dem Sinne "nachgiebiger" zu verstehen, und bei Bedarf kann auch die Innenhülse aus einem recht harten Material bestehen, vorausgesetzt, die erwünschte Dichtfunktion ist sichergestellt.

Gemäß einer besonders günstigen Ausgestaltung der erfindungsgemäßen Applikationsvorrichtung ist das Verformungselement oder der Verformungskörper seitlich an der Innenwand der Außenhülse angebracht und erstreckt sich mit zum Boden der Innenhülse hin zunehmender Tiefe in radialer Richtung spitz zulaufend. Die radiale Tiefe des Verformungselements nimmt zum Boden der Außenhülse hin kontinuierlich zu und beträgt der Innenhülse benachbart zunächst nahezu Null. Hierdurch ist der Einschubwiderstand der Innenhülse recht gering, und die Verformung erfolgt allmählich, während die Innenhülse eingeschoben wird.

Das Verformungselement kann entweder an seiner Oberseite ballig ausgebildet sein oder eine Schneide aufweisen. Bei der Ausgestaltung mit der Schneide ist es auch möglich, die harten Materialeigenschaften der Außenhülse so auszunutzen, dass der Boden der Innenhülse oder insbesondere ein oder mehrere Dichtringe regelrecht angeschnitten werden. Bei dieser Lösung sind dann Öffnungen in der Innenhülse im Grunde entbehrlich, wobei es sich versteht, dass beliebige geeignete Verformungselemente eingesetzt werden können, um eine Flüssigkeitsverbindung zwischen dem Innenraum der Innenhülse und dem Innenraum der Außenhülse herzustellen.

Es ist auch möglich, den Dichtring als einen in einer Nut an der Innenhülse geführten O-Ring auszubilden. Bei dieser Lösung kann dann die Schneide den O-Ring zerschneiden. Er zieht sich zusammen und gibt einen Bereich frei, in dem das Fluid über Öffnungen in den Innenraum der Innehülse strömen kann.

Es versteht sich, daß die Nut dann so gestaltet sein sollte, daß sie die Schneidwirkung der Schneide nicht behindert, sondern eher erleichtert.

Auch wenn die Realisierung eines asymmetrischen Verformungselements bevorzugt ist, ist es ohne weiteres auch möglich, zwei einander gegenüberliegende Verformungskörper vorzusehen, die die Innenhülse einquetschen und so den Spalt als Überlaufkanal zwischen dem Innenraum der Innenhülse und dem Innenraum der Außenhülse erzeugen. Es ist ohne weiteres möglich, den Verformungskörper in beliebiger geeigneter Weise auszugestalten, wobei sich bevorzugt unmittelbar neben ihm eine Innenecke erstreckt, die ein Überströmen an dem Boden der Innenhülse entlang erlaubt.

Das Applikationselement weist einen Benetzungskörper auf, der bei Bedarf entweder bereits vorab mit einer Reaktionssubstanz versehen ist, oder vor der Applikation mit einer Reaktionssubstanz versehen wird. Der Benetzungskörper kann in beliebiger geeigneter Weise ausgebildet sein, beispielsweise als Pinsel oder als Schaumkörper, der auf einem Stil des Applikationselements aufgebracht ist.

Es ist auch möglich, das Applikationselement selbst separat zu liefern und so eine unterschiedliche Kombination von Bestandteilen für die Bereitstellung der Substanz zu ermöglichen, wobei die Tatsache ausnutzbar ist, dass die Applikationsvorrichtung auch ohne Applikationselement abdichtet.

Weitere Vorteile, Einzelheiten und Merkmale ergeben sich aus der nachfolgenden Beschreibung eines Ausführungsbeispiels anhand der Zeichnung.

Es zeigen:
- Fig. 1: eine etwas schematisierte Ansicht einer Ausführungsform einer erfindungsgemäßen Applikationsvorrichtung, wobei das Applikationselement weggelassen ist und wobei die Innenhülse sich gegenüber der Außenhülse im Ausgangszustand befindet;
- Fig. 2: die Ausführungsform gemäß Fig. 1, wobei das Applikationselement eingesetzt ist;
- Fig. 3: die Ausführungsform gemäß Fig. 1 und 2, wobei auf das Applikationselement bereits Druck ausgeübt ist und die Innenhülse in die Außenhülse eingedrückt ist;
- Fig. 4: die Ausführungsform der Applikationsvorrichtung gemäß den Figuren 1 bis 3, wobei die Innenhülse vollständig in die Außenhülse eingetaucht und der Benetzungskörper des Applikationselements mit der Substanz benetzt ist;
- Fig. 5: einen Schnitt durch ein erfingungsgemäßes Verformungselement in der Anordnung in der Außenhülse; und
- Fig. 6: eine modifizierte Ausgestaltung einer erfindungsgemäßen Applikationsvorrichtung.

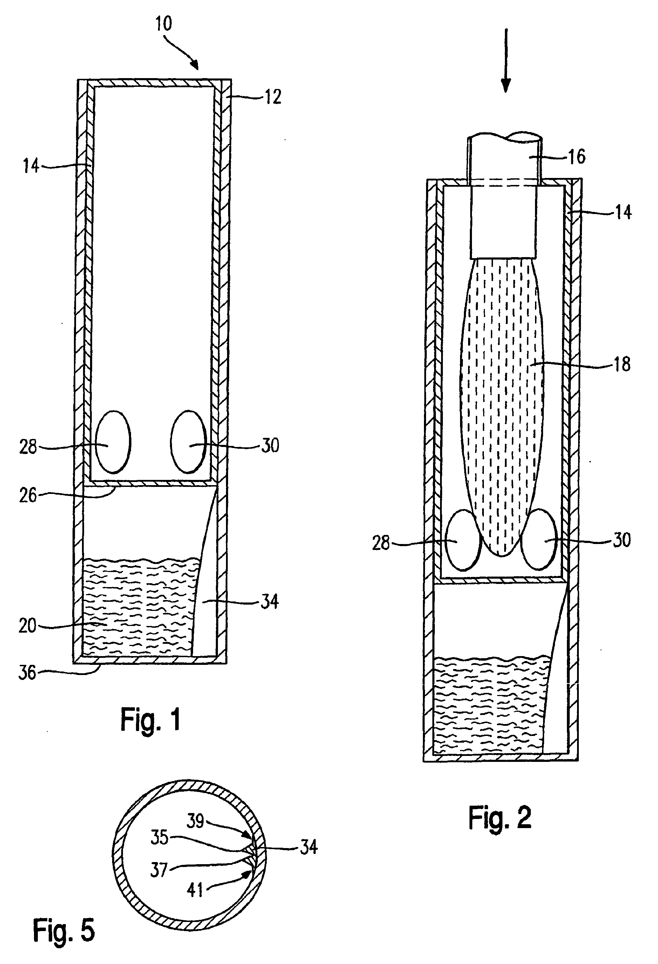

Die in Fig. 1 dargestellte Applikationsvorrichtung 10 weist eine Außenhülse 12 auf, die ein Gehäuse bildet. In der im wesentlichen becherförmigen Außenhülse ist eine Innenhülse 14 geführt, die erfindungsgemäß in besonderer Weise ausgestattet ist. Die Innenhülse 14 ist dafür bestimmt, ein Applikationselement 16 aufzunehmen, das aus Fig. 2 ersichtlich ist und einen Benetzungskörper 18 aufweist.

Die Außenhülse 12 ist länger als die Innenhülse 14 und ist in ihrem unteren Bereich mit einem Fluid 20 versehen, das für die Bereitstellung der Substanz vorgesehen ist.

Die Innenhülse 14 schließt so einen Bereich der Außenhülse, den Innenraum der Außenhülse 12, ab. Die Innenhülse 14 weist hierzu eine Dichtlippe 24 auf, die sich ringsum erstreckt und dem Boden 26 der Innenhülse 14 benachbart angeordnet ist. Die Innenhülse 14 ist in dem dargestellten Ausführungsbeispiel aus Kunststoff und die Außenhülse 12 ist in dem dargestellten Ausführungsbeispiel aus Glas. Die Dichtlippe 24 kann auf der glatten Innenwand der Außenhülse 12 gut abdichten.

Die Innenhülse 14 weist dem Boden 26 benachbart zwei ziemlich große Öffnungen 28 und 30 auf, die sich seitlich erstrecken. Diese Öffnungen sind von dem Fluid 20 bei eingedrückter Innenhülse 14 leicht und schnell durchströmbar.

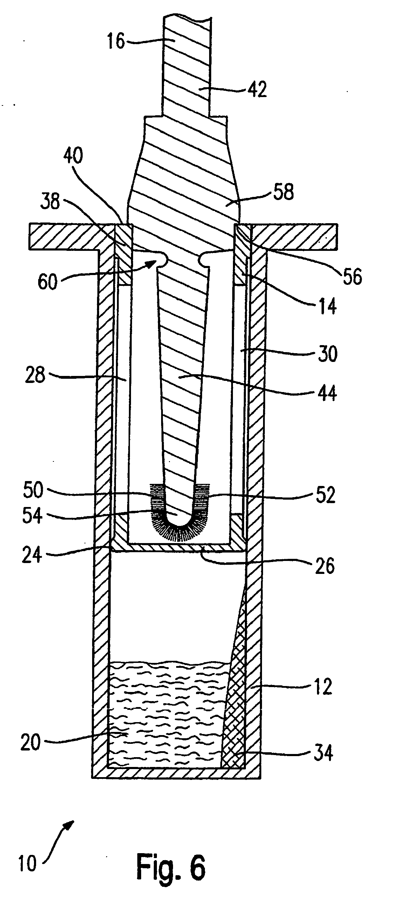

Zur Erzeugung einer Flüssigkeitsverbindung zwischen dem Innenraum der Innenhülse 14 und dem Innenraum der Außenhülse 12 dient erfindungsgemäß ein Verformungselement 34. Das Verformungselement 34 ist in dem dargestellten Ausführungsbeispiel als Keil ausgebildet und weist die aus Fig. 5 ersichtliche Form auf. Der Keil erstreckt sich vom Boden 26 der Innenhülse 14 in dem Zustand gemäß Fig. 1 ausgehend bis zum Boden 36 der Außenhülse 12.

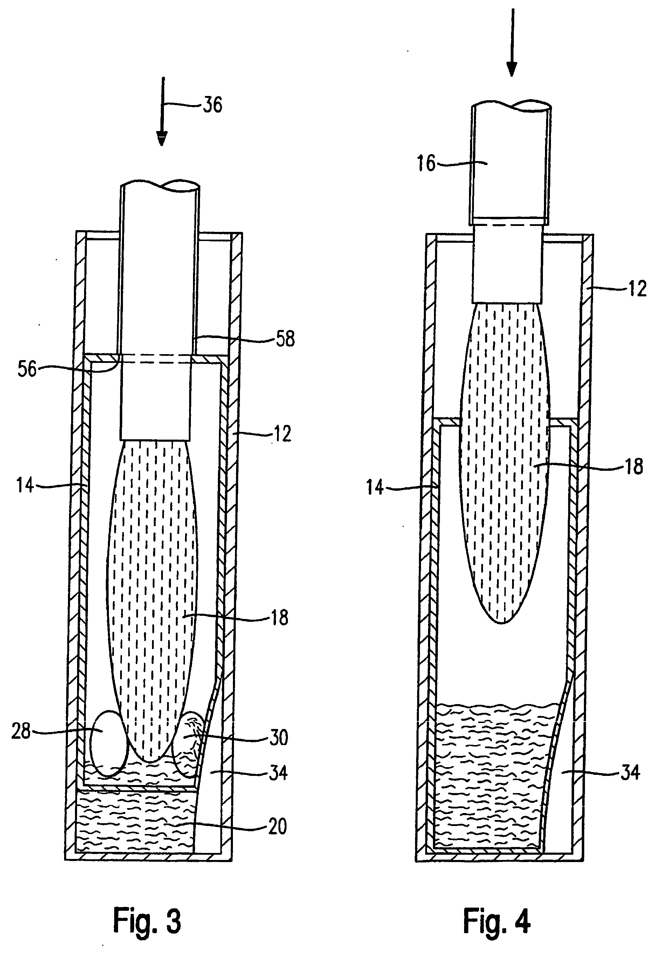

Während aus Fig. 2 ersichtlich ist, in welcher Weise das Applikationselement 16 mit dem Benetzungskörper 18 in die Innenhülse eingesetzt ist, zeigt Fig. 3 das Überströmen des Fluids 20 in die Innenhülse 14 hinein. Durch Druck in Richtung des Keils 36 gemäß Fig. 3 auf das Applikationselement 16 wird zugleich die Innenhülse 14 eingedrückt. Hierzu weist die Innenhülse eine Anschlagschulter 56 auf, die sich mit einem Durchmesser erstreckt, der etwas geringer als der Durchmesser einer Verdickung 58 des Applikationselements 16 ist. Das Ausüben von Druck ist auf diese Weise auf die Innenhülle 14 möglich, ohne dass eine Spitze 54 den Boden 26 berühren muß.

Aus Fig. 4 ist ersichtlich, dass der Benetzungskörper 18 von der Substanz 20 intensiv benetzt ist und die Substanz 20 über das Applikationselement 16 aufgetragen werden kann. In diesem Zustand bildet die Applikationsvorrichtung 10 eine Art Becher für das Auftragen der Substanz, wobei auch ein mehrmaliges Eintauchen des Benetzungskörpers 18 ohne weiteres möglich ist.

Aus Fig. 5 ist ersichtlich, in welcher Weise das Verformungselement 34 ausgebildet sein kann. In dem dargestellten Ausführungsbeispiel ist es im wesentlichen keilförmig mit zwei Spitzen 35 und 37, die einen geringen Gleitwiderstand beim Aufgleiten des Bodens 26 auf das Verformungselement 34 ermöglichen. Es ist ersichtlich, dass seitlich neben dem Verformungselement Innenecken 39 und 41 vorgesehen sind, die ein leichtes Durchströmen der Substanz in den Innenraum der Innenhülse 14 hinein ermöglichen.

Aus Fig. 6 ist eine weitere Ausführungsform der erfindungsgemäßen Applikationsvorrichtung ersichtlich. In dieser Ausführungsform ist ersichtlich, dass die Innenhülse 14 einen Abschlussring 38 aufweist, an dem sich ein Vorsprung 40 nach außen erstreckt. Dort ist eine Dichtlippe ausgebildet, die bei eingesetztem Applikationselement 16 eine Abdichtung gewährleistet.

Das Applikationselement 16 weist einen Griff 42 auf. Der Griff schließt sich an eine Verdickung 58 an, an die sich der Schaft 44 anschließt. In diesem Ausführungsbeispiel ist der Benetzungskörper als Mikropinsel 50 ausgebildet. Der Mikropinsel 50 ist in dem dargestellten Ausführungsbeispiel durch eine Beflockung gebildet, so dass sich zahlreiche wirr angeordnete Pinselhaare 52 nach außen erstrecken.

Der Mikropinsel 50 endet in einer eher stumpfen Spitze 54. Diese ermöglicht auch die Kraftübertragung auf den Boden 26 der engen Hülse 14 und damit das Einschieben der Innenhülse 14 zur Aktivierung der Applikationsvorrichtung, wenn die Anlageschulter 56 nicht vorgesehen ist.

Auf dem Mikropinsel 50 kann bei Bedarf eine Reaktionssubstanz bevorzugt als dort abgelagertes Salz aufgebracht sein. Bei dieser Ausführungsform ist unterhalb der Anlageschulter 56 eine Verjüngung 60 oder Kerbe vorgesehen, die die Möglichkeit bietet, den Schaft 44 gegenüber dem Griff 42 abzubiegen. Bei dieser Konfiguration des Applikationselements ist eine plastische Verformung möglich. Auch hier ist es möglich, den Boden 26 über das Verformungselement 34 zu verformen, um ein Benetzen des Mikropinsels 50 mit der Substanz 20 einzuleiten.

## Patentansprüche

1. Applikationsvorrichtung für das Auftragen einer Substanz auf einen Applikationsort, wobei ein Applikationselement mit einem Benetzungskörper in eine Innenhülse teilweise aufgenommen und die Innenhülse in eine Außenhülse aufgenommen ist und wobei die Innenhülse (14) insbesondere aus einem weicheren Material als die Außenhülse (12) besteht, **dadurch gekennzeichnet, dass** durch Druck auf das Applikationselement eine Flüssigkeitsverbindung zwischen dem Innenraum der Innenhülse und dem Innenraum der Außenhülse erzeugbar ist, und beim Eindrücken mindestens der Boden (26) der Innenhülse (14) verformbar ist oder der bzw. die Dichtringe (24) im Bereich des Bodens (26) der Innenhülse (14) zerschnitten werden und dass der Boden (26) der Innenhülse (14) unter Erzeugung eines Spalts zwischen der Innenhülse (14) und der Aussenhülse (12) verformbar ist.

2. Applikationsvorrichtung nach Anspruch 1, **dadurch gekennzeichnet, dass** der Boden (26) der Innenhülse (14) beim Eindrücken des Applikationselements (16) bevorzugt asymmetrisch verformbar ist.

3. Applikationsvorrichtung nach einem der vorhergehenden Ansprüche, **dadurch gekennzeichnet, dass** die Innenhülse (14) dem Boden (26) benachbart seitliche Öffnungen (28, 30) aufweist, und dass die Wand der Innenhülse (14) mindestens bis zu den Öffnungen verformbar ist.

4. Applikationsvorrichtung nach einem der vorhergehenden Ansprüche, **dadurch gekennzeichnet, dass** in der Aussenhülse (12) ein oder mehrere Verformungskörper (34) angebracht sind, die beim Eindrücken des Applikationselements (16) die Innenhülse (14) verformen.

5. Applikationsvorrichtung nach einem der vorhergehenden Ansprüche, **dadurch gekennzeichnet, dass** der oder die Verformungskörper (34) dem Ende der Aussenhülse (12) benachbart vorzugsweise einseitig in der Aussenhülse (12) angebracht sind.

6. Applikationsvorrichtung nach einem der vorhergehenden Ansprüche, **dadurch gekennzeichnet, dass** ein oder mehrere Verformungskörper für das Verformen des Bodens (26) der Innenhülse (14) eine Schrägfläche aufweisen, die sich von der Wand der Aussenhülse (12) schräg zur Innenhülse (14) hin erstreckt.

7. Applikationsvorrichtung nach einem der vorhergehenden Ansprüche, **dadurch gekennzeichnet, dass** ein oder mehrere Verformungskörper dem Ende der Aussenhülse (12) benachbart in dieser angebracht sind und einen in wesentlichen keilförmigen Aufbau aufweisen.

8. Applikationsvorrichtung nach einem der vorhergehenden Ansprüche, **dadurch gekennzeichnet, dass** ein Verformungskörper für die Verformung des der Boden (26) der Innenhülse (14) in der Aussenhülse (12) angebracht ist und mindestens eine der Innenhülse (14) zugewandte Schneide aufweist.

9. Applikationsvorrichtung nach einem der vorhergehenden Ansprüche, **dadurch gekennzeichnet, dass** ein oder mehrere Verformungskörper (34) in der Aussenhülse (12) angebracht sind und sich vor dem Eindrücken des Applikationselements (16) von der Innenhülse (14) beabstandet oder höchstens an diese anliegend erstrecken.

10. Applikationsvorrichtung nach einem der vorhergehenden Ansprüche, **dadurch gekennzeichnet, dass** die Aussenhülse (12) aus einem hochdichten Material wie Barex, Topas, Glas und/oder Metall besteht.

11. Applikationsvorrichtung nach einem der vorhergehenden Ansprüche, **dadurch gekennzeichnet, dass** dem Boden (26) der Innenhülse (14) benachbart eine Dichtlippe sich nach aussen erstreckt.

12. Applikationsvorrichtung nach einem der vorhergehenden Ansprüche, **dadurch gekennzeichnet, dass** die Dichtlippe (24) für das Abdichten des Innenraums der Aussenhülse (12) von dem Innenraum der Innenhülse (14) bevorzugt einstückig an die Innenhülse (14) angeformt ist.

13. Applikationsvorrichtung nach einem der vorhergehenden Ansprüche, **dadurch gekennzeichnet, dass** die Dichtlippe (24) für das Abdichten des Innenraums der Aussenhülse (12) von dem der Innenhülse (14) von dem Verformungskörper (34) mindestens verformbar, insbesondere zerstörbar ist.

14. Applikationsvorrichtung nach einem der vorhergehenden Ansprüche, **dadurch gekennzeichnet, dass** die Dichtlippe (24) für das Abdichten des Innenraums der Aussenhülse (12) von dem der Innenhülse (14) als unter Vorspannung stehender O-Ring ausgebildet ist, der beim Zerschneiden durch den Verformungskörper (34) ein Durchströmen des Fluids ermöglicht.

## Claims

1. An applicator device for applying a substance to an application site, wherein an applicator element with a wetting body is partly accommodated in an inner sleeve and the inner sleeve is accommodated in an outer sleeve, and wherein the inner sleeve (14) in particular consists of a softer material than the outer sleeve (12), **characterised in that** by applying pressure on to the applicator element a fluid connection can be established between the inner chamber of the inner sleeve (14) and the inner chamber of the outer sleeve (12), and upon being pressed in at least the base (26) of the inner sleeve (14) can be deformed or the sealing ring or rings (24) in the vicinity of the base (26) of the inner sleeve (14) is/are cut, and **in that** the base (26) of the inner sleeve (14) can be deformed thus creating a clearance between the inner sleeve (14) and the outer sleeve (12).

2. An applicator device according to Claim 1, **characterised in that** the base (26) of the inner sleeve (14) can be deformed preferably asymmetrically when the applicator element (16) is pressed in.

3. An applicator device according to either one of the preceding Claims, **characterised in that** the inner sleeve (14) has lateral openings (28,30) adjacent the base (26), and **in that** the wall of the inner sleeve (14) can be deformed at least up to the openings.

4. An applicator device according to any one of the preceding Claims, **characterised in that** the one or more deformation bodies (34) are provided in the outer sleeve (12), which bodies deform the inner sleeve (14) when the applicator element (16) is pressed in.

5. An applicator device according to any one of the preceding Claims, **characterised in that** the deformation body or bodies (34) is/are provided adjacent the end of the outer sleeve (12), preferably on one side of the outer sleeve (12).

6. An applicator device according to any one of the preceding Claims, **characterised in that** one or more deformation bodies for deforming the base (26) of the inner sleeve (14) have an inclined surface which extends from the wall of the outer sleeve (12) obliquely towards the inner sleeve (14).

7. An applicator device according to any one of the preceding Claims, **characterised in that** one or more deformation bodies are provided in the outer sleeve (12) adjacent the end thereof and are of substantially wedge-shaped configuration.

8. An applicator device according to any one of the preceding Claims, **characterised in that** one deformation body for deforming [sic] the base (26) of the inner sleeve (14) is provided in the outer sleeve (12) and has at least one cutting edge facing the inner sleeve (14).

9. An applicator device according to any one of Claims, **characterised in that** one or more deformation bodies are provided in the outer sleeve (12) and before the pressing-in of the applicator element (16) are spaced apart from the inner sleeve (14) or at most extend abutting on the latter.

10. An applicator device according to any one of the preceding Claims, **characterised in that** the outer sleeve (12) consists of a high-density material, such as Barex, topaz, glass and/or metal.

11. An applicator device according to any one of the preceding Claims, **characterised in that** a sealing lip extends outwards adjacent the base (26) of the inner sleeve (14).

12. An applicator device according to any one of the preceding Claims, **characterised in that** the sealing lip (24) for sealing the inner chamber of the outer sleeve (12) from the inner chamber of the inner sleeve (14) is preferably formed integrally on the inner sleeve (14).

13. An applicator device according to any one of the preceding Claims, **characterised in that** the sealing lip (24) for sealing the inner chamber of the outer sleeve (12) from that of the inner sleeve (14) can at least be deformed by the deformation body (34), in particular can be destroyed thereby.

14. An applicator device according to any one of the preceding Claims, **characterised in that** the sealing lip (24) for sealing the inner chamber of the outer sleeve (12) from that of the inner sleeve (14) is in the form of an O-ring under preloading, which enables fluid to flow through upon being cut by the deformation body (34).

## Revendications

1. Dispositif d'application pour l'application d'une substance à un emplacement déterminé, un élément applicateur avec un corps de mouillage étant logé partiellement dans une gaine intérieure et la gaine intérieure étant logée dans une gaine extérieure, et la gaine intérieure (14) étant réalisée en particulier dans un matériau plus mou que celui de la gaine extérieure (12), **caractérisé en ce qu'**une liaison d'écoulement peut s'établir entre le volume intérieur de la gaine intérieure (14) et le volume intérieur de la gaine extérieure (12) sous l'effet de la pression exercée sur l'élément applicateur, et lors de l'enfoncement de celui-ci au moins le fond (26) de la gaine intérieure (14) est déformable ou la ou les bagues d'étanchéité (24) dans la zone du fond (26) de la gaine intérieure (14) sont sectionnées, et **en ce que** le fond (26) de la gaine intérieure (14) est déformable moyennant la formation d'une fente entre la gaine intérieure (14) et la gaine extérieure (12).

2. Dispositif d'application selon la revendication 1, **caractérisé en ce que**, lors de l'enfoncement de l'élément applicateur (16), le fond (26) de la gaine intérieure (14) est apte à se déformer, de préférence asymétriquement.

3. Dispositif d'application selon l'une quelconque des revendications précédentes, **caractérisé en ce que** la gaine intérieure (14) comporte des orifices (28, 30) latéraux, adjacents au fond (26), et **en ce que** la paroi de la gaine intérieure (14) est déformable au moins jusqu'aux orifices.

4. Dispositif d'application selon l'une quelconque des revendications précédentes, **caractérisé en ce qu'**un ou plusieurs corps de déformation (34) sont disposés dans la gaine extérieure (12), lesquels déforment la gaine intérieure (14) lors de l'enfoncement de l'élément applicateur (16).

5. Dispositif d'application selon l'une quelconque des revendications précédentes, **caractérisé en ce que** le ou les corps de déformation (34) sont disposés dans la gaine extérieure (12) en étant adjacents à l'extrémité de la gaine extérieure (12), de préférence sur un côté de celle-ci.

6. Dispositif d'application selon l'une quelconque des revendications précédentes, **caractérisé en ce qu'**un ou plusieurs corps de déformation destinés à déformer le fond (26) de la gaine intérieure (14) ont une face oblique, qui s'étend en oblique depuis la paroi de la gaine extérieure (12) vers la gaine intérieure (14).

7. Dispositif d'application selon l'une quelconque des revendications précédentes, **caractérisé en ce qu'**un ou plusieurs corps de déformation, adjacents à l'extrémité de la gaine extérieure (12), sont logés dans celle-ci et ont une structure sensiblement en forme de cale.

8. Dispositif d'application selon l'une quelconque des revendications précédentes, **caractérisé en ce qu'**un corps de déformation destiné à déformer le fond (26) de la gaine intérieure (14) est disposé dans la gaine extérieure (12) et comporte au moins une arête de coupe orientée vers la gaine intérieure (14).

9. Dispositif d'application selon l'une quelconque des revendications précédentes, **caractérisé en ce qu'**un ou plusieurs corps de déformation (34) sont disposés dans la gaine extérieure (12) et, avant l'enfoncement de l'élément applicateur (16), sont écartés de la gaine intérieure (14) ou, au pire, sont en appui contre celle-ci.

10. Dispositif d'application selon l'une quelconque des revendications précédentes, **caractérisé en ce que** la gaine extérieure (12) est réalisée dans un matériau très dense, tel que le barex, la topaze, le verre et/ou le métal.

11. Dispositif d'application selon l'une quelconque des revendications précédentes, **caractérisé en ce qu'**une lèvre d'étanchéité, adjacente au fond (26) de la gaine intérieure (14), s'étend vers l'extérieur.

12. Dispositif d'application selon l'une quelconque des revendications précédentes, **caractérisé en ce que** la lèvre d'étanchéité (24), destinée à étancher le volume intérieur de la gaine extérieure (12) par rapport au volume intérieur de la gaine intérieure (14), est formée de préférence d'un seul tenant sur la gaine intérieure (14).

13. Dispositif d'application selon l'une quelconque des revendications précédentes, **caractérisé en ce que** la lèvre d'étanchéité (24), destinée à étancher le volume intérieur de la gaine extérieure (12) par rapport à celui de la gaine intérieure (14), peut être au moins déformée, en particulier abîmée par le corps de déformation (34).

14. Dispositif d'application selon l'une quelconque des revendications précédentes, **caractérisé en ce que** la lèvre d'étanchéité (24), destinée à étancher le volume intérieur de la gaine extérieure (12) par rapport à celui de la gaine intérieure (14), est réalisée sous forme de joint torique sous précontrainte qui, lors du sectionnement par le corps de déformation (34), permet un passage du fluide.
